# EUROPEAN PATENT APPLICATION

(11) **EP 3 143 921 A1**
(43) Date of publication of application: **22.03.2017**
(21) Application number: 15793298.9
(22) Date of filing: 17.04.2015
(51) Int. Cl.: A61B 1/00, A61B 17/00

(54) **ADAPTER FOR INSTRUMENT AND MANIPULATOR SYSTEM FOR OPERATION**

(30) Priority: 15.05.2014 JP 2014101386
(71) Applicant: OLYMPUS CORPORATION, Hachioji-shi Tokyo 192-8507 (JP)
(72) Inventor: YOSHII, Toshihiro, Tokyo 192-8507 (JP)
(74) Representative: Schicker, Silvia
(86) International application number: PCT/JP2015/061859
(87) International publication number: WO 2015/174213

(57) **Abstract**

This treatment instrument adaptor allows a treatment instrument to be rotated about the longitudinal axis while an insertion portion is placed in the body. This treatment instrument adaptor (6) has a cap member (1) attached to a distal end of an insertion portion (5) having a channel and that has a through hole aligned with an opening of the channel; a tubular fitting member attached to the through hole of the cap member so as to be rotatable about an axis of the through hole and so as not to be movable along the axis and that has an inner hole into which a treatment instrument (8) is fitted and with which the treatment instrument is engaged in a circumferential direction; and a rotating mechanism (14) including a driving-force transmitting member (15) that is wound around the fitting member and that rotates the fitting member about the axis using tension.

## Description

### {Technical Field}

The present invention relates to treatment instrument adaptors and surgical manipulator systems.

### {Background Art}

There is a known endoscope having a tube or treatment instrument detachably secured to the distal end of an insertion portion by a holder (see, for example, PTL 1).

This endoscope has a groove extending radially along the longitudinal axis direction at the distal end of a channel provided in the insertion portion. A tube or treatment instrument extending along the groove can be pulled into the groove and secured by a holder guided through the channel, such as snare forceps.

Since the treatment instrument is secured to the insertion portion, when the treatment instrument has a bended portion at the distal end thereof, the bending direction of the treatment instrument can be determined in advance relative to the angle of view of the endoscope. Thus, it is advantageous that the operator can determine, in advance, direction to which the treatment instrument bends when manipulating the treatment instrument, watching an endoscopic image displayed on a monitor.

### {Citation List}

### {Patent Literature}

{PTL 1}
Japanese Unexamined Patent Application, Publication No. 2002-200034

### {Summary of Invention}

### {Technical Problem}

However, since the endoscope in PTL 1 uses snare forceps as a holder to secure the treatment instrument to the insertion portion, it is difficult to adjust the bending direction of the treatment instrument relative to the insertion portion while the insertion portion and the treatment instrument are placed in the body. Specifically, since a thin treatment instrument transmits little torsion, a torsion applied to the treatment instrument by the operator positioned outside the body is absorbed by the elongated treatment instrument in the channel. Therefore, it is difficult to rotate the distal end of the treatment instrument about the longitudinal axis thereof.

Thus, when the bending direction of the treatment instrument should be changed relative to the angle of view of the endoscope after insertion into the body, for example, when the bending direction is shifted for some reason during manipulation or when the bending direction is needed to be changed for facilitating treatment, the insertion portion and the treatment instrument have to be removed from the body to perform adjustments outside the body, which is time-consuming.

The present invention is made considering the aforementioned circumstances, and an object of the present invention is to provide a treatment instrument adaptor and surgical manipulator system that allow a treatment instrument to be rotated about the longitudinal axis thereof for improved manipulation while an insertion portion is placed in the body.

### {Solution to Problem}

To achieve the foregoing object, the present invention provides the following solutions.

An aspect of the present invention provides a treatment instrument adaptor comprising: a cap member that is attached to a distal end of a flexible insertion portion having a channel and that has a through hole located at a position which is aligned with an opening of the channel; a substantially tubular fitting member that is attached to the through hole of the cap member so as to be rotatable about an axis of the through hole and so as not to be movable along the axis and that has an inner hole into which a treatment instrument guided through the channel is fitted and with which the treatment instrument is engaged in a circumferential direction; and a rotating mechanism including a driving-force transmitting member that is wound around the fitting member and that rotates the fitting member about the axis using tension.

According to this aspect, when the cap member is attached to the distal end of the insertion portion, the through hole of the cap member is aligned with the opening of the channel of the insertion portion. The treatment instrument guided through the channel is moved out of the opening of the channel and then protrudes from the cap member through the through hole of the cap member. Since the tubular fitting member is located in the through hole, when the treatment instrument is moved through the through hole, the treatment instrument is fitted into the inner hole of the tubular fitting member and is engaged with the fitting member in the circumferential direction.

In this state, the rotating mechanism is operated to rotate the fitting member about the axis thereof by applying tension to the driving-force transmitting member wound around the fitting member. This allows the rotating force around the axis to be transmitted to the treatment instrument engaged with the fitting member in the circumferential direction. Whereas an elongated treatment instrument can transmit little rotating force to the distal end thereof when twisted at the proximal end side of the insertion portion, the driving-force transmitting member can directly transmit a rotating force to the vicinity of the distal end. This allows the treatment instrument to be easily and reliably rotated about the axis thereof.

In the above aspect, the fitting member may be made of an elastic material that allows an inner diameter of the inner hole to be changed, and the inner diameter of the inner hole may be decreased to engage the inner hole with the treatment instrument in a state in which the treatment instrument is placed in the inner hole.

In this case, the inner diameter of the inner hole of the fitting member may be increased to facilitate the insertion of the treatment instrument into the inner hole of the fitting member, and the inner diameter of the inner hole of the fitting member may be decreased to engage the treatment instrument with the fitting member in the circumferential direction after the insertion of the treatment instrument.

In the above aspect, the fitting member may have a C-shaped cross-section with a slit provided at a part of the circumferential direction.

In this case, the inner diameter of the inner hole can be decreased by narrowing the slit and can be increased by widening the slit.

In the above aspect, the treatment instrument adaptor may further include a tightening mechanism that applies tension to the driving-force transmitting member to decrease the inner diameter of the inner hole of the fitting member.

In this case, when the tension is applied to the driving-force transmitting member, which is wound around the fitting member, for example, one or more times, the driving-force transmitting member is tightened around the fitting member to compress the fitting member against its elastic restoring force. This makes the inner diameter of the inner hole decrease to engage the treatment instrument in the circumferential direction by friction.

In the above aspect, the treatment instrument adaptor may further include a fixed member fixed to the insertion portion and a moving mechanism that moves the cap member relative to the fixed member in a longitudinal axis direction of the insertion portion.

In this case, the treatment instrument also engages with the fitting member in the longitudinal axis direction by friction because the fitting member is compressed and engaged with the treatment instrument. Thus, the moving mechanism can be operated to move the cap member relative to the fixed member fixed to the insertion portion in the longitudinal axis direction of the insertion portion while the fitting member is compressed and engaged with the treatment instrument. This allows the position of the treatment instrument engaged with the fitting portion of the cap member to be adjusted in the longitudinal axis direction.

In the above aspect, the treatment instrument may be provided with a pillar-like fitting portion having a noncircular cross-section, and the inner hole of the fitting member has a cross-section that allows the pillar-like fitting portion to be fitted into the inner hole and to be engaged with the inner hole of the fitting member in the circumferential direction.

In this case, the treatment instrument can be engaged with the fitting member in the circumferential direction simply by fitting the pillar-like fitting portion, which has a noncircular cross-section, into the inner hole of the fitting member in the axial direction.

In the above aspect, a surgical manipulator system is provided that includes any of the above treatment instrument adaptors, an endoscope to which the cap member of the treatment instrument adaptor is attached at a distal end of the endoscope, and a treatment instrument that is guided through the channel of the insertion portion of the endoscope and that includes an end effector that is protruded from the distal end of the insertion portion through the inner hole of the fitting member.

### {Advantageous Effects of Invention}

The present invention affords an advantage of allowing a treatment instrument to be rotated about the longitudinal axis thereof for improved manipulation while an insertion portion is placed in the body.

### {Brief Description of Drawings}

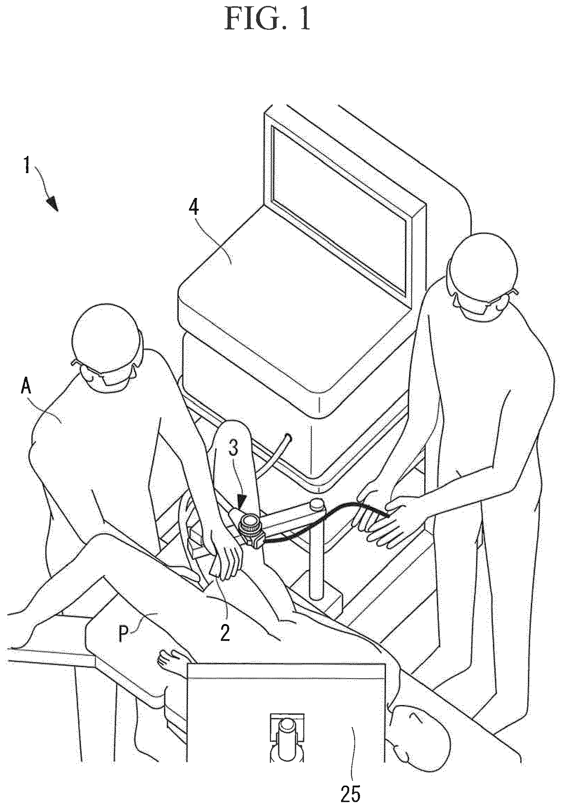

{Fig. 1}
   Fig. 1 is an overall view showing a surgical manipulator system according to an embodiment of the present invention.
{Fig. 2}
   Fig. 2 is a perspective view showing a treatment instrument adaptor, according to the embodiment of the present invention, that is attached to an insertion portion of an endoscope provided in the surgical manipulator system in Fig. 1.
{Fig. 3}
   Fig. 3 is an enlarged perspective view showing the distal end of the endoscope equipped with the treatment instrument adaptor in Fig. 2.
{Fig. 4A}
   Fig. 4A is a partially cutaway front view showing a release position of a tightening mechanism of the treatment instrument adaptor in Fig. 2.
{Fig. 4B}
   Fig. 4B is a partially cutaway front view showing a lock position of the tightening mechanism of the treatment instrument adaptor in Fig. 2.
{Fig. 5A}
   Fig. 5A is a perspective view showing a release position of an example fitting member for the treatment instrument adaptor in Fig. 2.
{Fig. 5B}
   Fig. 5B is a perspective view showing a lock position of the example fitting member for the treatment instrument adaptor in Fig. 2.
{Fig. 6A}
   Fig. 6A is a longitudinal sectional view showing a release position of the treatment instrument adaptor in Fig. 2.
{Fig. 6B}
   Fig. 6B is a longitudinal sectional view showing a lock position of the treatment instrument adaptor in Fig. 2.
{Fig. 7A}
   Fig. 7A shows the relationship between the fitting member and the treatment instrument in Fig. 5 in the release position, as viewed in the axial direction.
{Fig. 7B}
   Fig. 7B shows the relationship between the fitting member and the treatment instrument in Fig. 5 in the lock position, as viewed in the axial direction.
{Fig. 7C}
   Fig. 7C shows the relationship between the fitting member and the treatment instrument in Fig. 5 during rotation, as viewed in the axial direction.
{Fig. 8}
   Fig. 8 is a perspective view showing a fitting member of a first modification of the treatment instrument adaptor in Fig. 2.
{Fig. 9}
   Fig. 9 shows the relationship between the fitting member and the treatment instrument in Fig. 8 in the release position, as viewed in the axial direction.
{Fig. 10A}
   Fig. 10A is a longitudinal sectional view showing a second modification of the treatment instrument adaptor in Fig. 2, with the treatment instrument moved backward in the axial direction.
{Fig. 10B}
   Fig. 10B is a longitudinal sectional view showing the second modification of the treatment instrument adaptor in Fig. 2, with the treatment instrument moved forward in the axial direction.
{Fig. 11A}
   Fig. 11A is a longitudinal sectional view showing a third modification of the treatment instrument adaptor in Fig. 2, with the treatment instrument moved backward in the axial direction.
{Fig. 11B}
   Fig. 11B is a longitudinal sectional view showing the third modification of the treatment instrument adaptor in Fig. 2, with the treatment instrument moved forward in the axial direction.

### {Description of Embodiments}

A treatment instrument adaptor and surgical manipulator system according to an embodiment of the present invention will be described below with reference to the drawings.

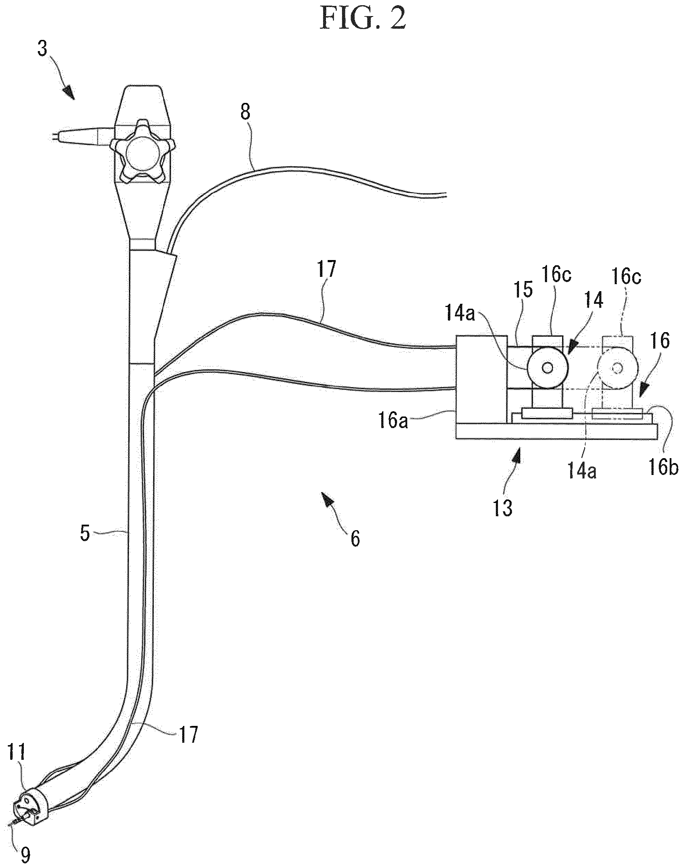

As shown in Fig. 1, the surgical manipulator system according to this embodiment is an endoscope system 1 including a manipulation device 2 that is manipulated by a doctor (operator) A, an endoscope 3 that is driven by inputs via the manipulation device 2, a controller 4 that controls the endoscope 3 based on the inputs using the manipulation device 2, a treatment instrument adaptor 6 (see Fig. 2) attached to the endoscope 3, and a monitor 25.

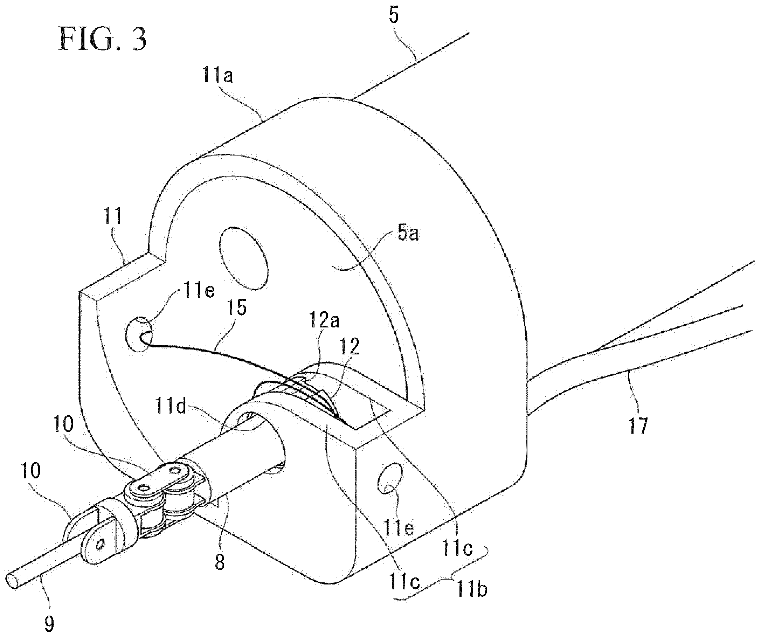

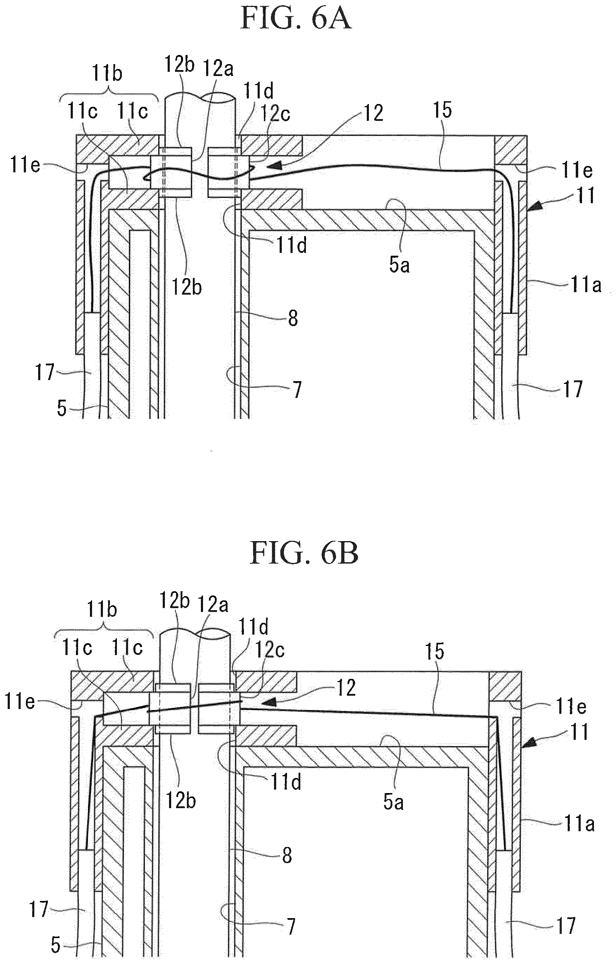

As shown in Fig. 2, the endoscope 3 includes an insertion portion 5 to be inserted into the body of a patient P and is equipped with the treatment instrument adaptor 6 according to this embodiment. As shown in Fig. 6, the insertion portion 5 of the endoscope 3 has a channel 7, extending in the longitudinal direction, through which an elongated treatment instrument is inserted throughout the length the channel, from the proximal to the distal end thereof. A treatment instrument 8 is inserted through the channel 7. For example, as shown in Fig. 3, the treatment instrument 8 includes two joints 10 that allow an end effector (e.g., energy forceps) 9 at the distal end thereof to pivot.

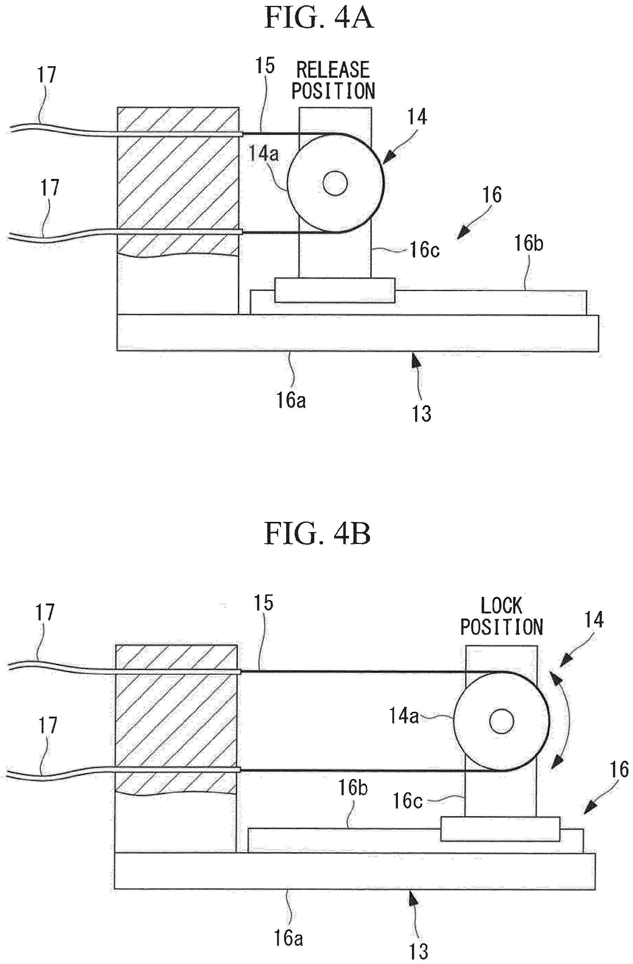

As shown in Fig. 3, the treatment instrument adaptor 6 includes a cap member 11 attached to the distal end of the insertion portion 5 and a fitting member 12 attached to the cap member 11. As shown in Figs. 2 and 4, the treatment instrument adaptor 6 further includes a tightening mechanism 13 that holds the treatment instrument 8 with the fitting member 12 and a rotating mechanism 14 that rotates the fitting member 12.

As shown in Figs. 3 and 6, the cap member 11 includes a tubular fitting portion 11a into which the distal end of the insertion portion 5 is fitted and an abutment portion 11b that is abutted by an tip surface 5a of the insertion portion 5 when the insertion portion 5 is fitted into the fitting portion 11a. The abutment portion 11b includes two plates 11c that are positioned in front of the tip surface 5a of the insertion portion 5 having a distance from each other in the longitudinal direction, when the distal end of the insertion portion 5 is fitted into the fitting portion 11a.

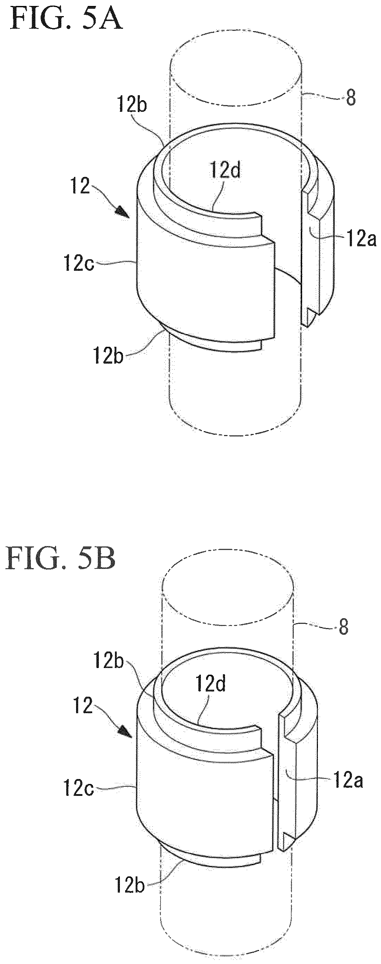

Each of the two plates 11c has a through hole 11d positioned to be aligned with the opening of the channel 7 provided in the insertion portion 5 when the cap member 11 is attached to the insertion portion 5. The through holes 11d have a slightly larger diameter than the channel 7. As shown in Figs. 5 and 6, the fitting member 12 is attached to the through holes 11d.

As shown in Fig. 5, the fitting member 12 is a substantially cylindrical member having a C-shaped cross-section with a slit 12a extending in the longitudinal direction. The fitting member 12 includes small-diameter portions 12b at the ends thereof in the longitudinal direction and a large-diameter portion 12c between the small-diameter portions 12b. The small-diameter portions 12b have an outer diameter smaller than the inner diameter of the through holes 11d of the cap member 11 when the fitting member 12 is in a free state. The large-diameter portion 12c has an outer diameter larger than the inner diameter of the through holes 11d and a height shorter than the distance between the plates 11c in the longitudinal direction when the fitting member 12 is in a free state. The fitting member 12 also has an inner hole 12d that has an inner diameter larger than the outer diameter of the treatment instrument 8 when the fitting member 12 is in a free state. When the fitting member 12 is compressed, the inner diameter of the fitting member 12 becomes smaller than the outer diameter of the treatment instrument 8.

Thus, the fitting member 12 is attached to the cap member 11 by fitting the two small-diameter portions 12b into the through holes 11d of the plates 11c and holding the large-diameter portion 12c between the plates 11c in the axial direction so that the fitting member 12 can be rotated about the axis of the through holes 11d and cannot be detached in the axial direction.

As shown in Figs. 3, 4, and 6, the tightening mechanism 13 includes a slider mechanism 16 that applies tension to a wire (driving-force transmitting member) 15 wound around the fitting member 12. As shown in Figs. 2, 3, and 6, the wire 15 has one end thereof secured to the cap member 11 and the other end thereof inserted into wire holes 11e provided in the cap member 11, and also through two sheaths 17 secured to a base member 16a of the slider mechanism 16. The wire holes 11e have openings located opposite each other, with the fitting member 12 attached to the cap member 11 disposed therebetween in the radial direction. The wire 15 goes through the openings and is wound around the fitting member 12 one or more times in the circumferential direction.

The slider mechanism 16 includes a base member 16a, a straight guide rail 16b secured to the base member 16a, and a slider 16c supported on the guide rail 16b so as to be linearly movable in the longitudinal direction thereof.

The rotating mechanism 14 includes the wire 15, a pulley 14a around which the wire 15 runs and that is supported on the slider 16c of the slider mechanism 16 so as to be rotatable about an axis perpendicular to the longitudinal direction of the guide rail 16b, and a motor (not shown) that rotates the pulley 14a about the axis thereof.

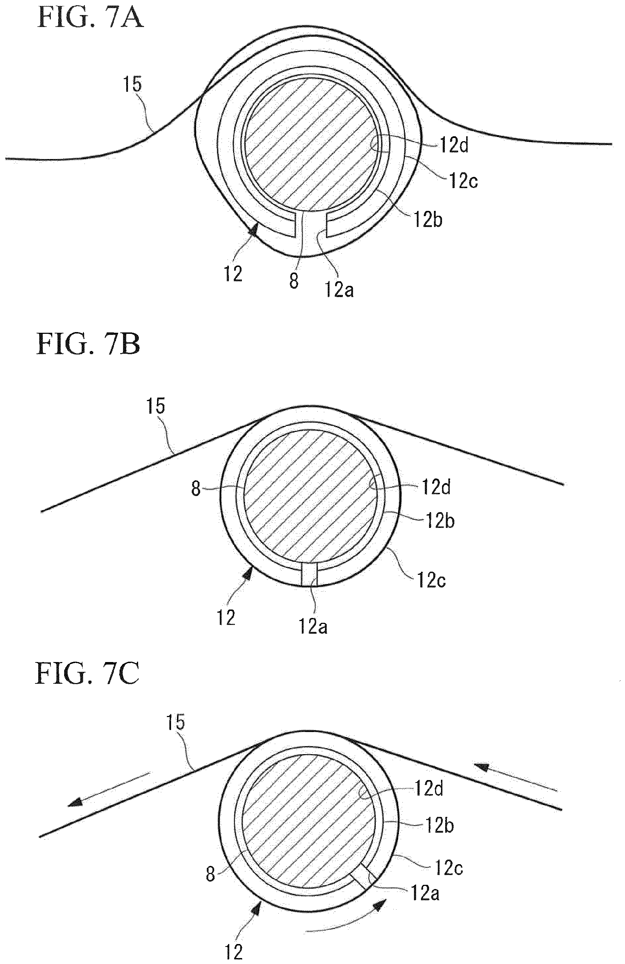

As shown in Figs. 6A and 7A, the wire 15 is loosened when the slider 16c of the slider mechanism 16 is located at a release position shown in Fig. 4A. As shown in Figs. 6B and 7B, the wire 15 is stretched under tension when the slider 16c is located at a lock position shown in Fig. 4B.

When the slider 16c is moved to the lock position, the wire 15 is tightened around the fitting member 12 under tension, and thereby the fitting member 12 is elastically deformed to decrease the inner diameter of the inner hole 12d as shown in Fig. 5B. When the slider 16c is moved to the release position, the wire 15 is loosened around the fitting member 12 to release the tightening force. As shown in Fig. 5A, the fitting member 12 is deformed by its elastically restoring force to increase the inner diameter of the inner hole 12d.

While the slider 16c is placed in the lock position, the pulley 14a of the rotating mechanism 14 is rotated by driving the motor to apply a tension corresponding to the rotational direction of the pulley 14a to the wire 15 wound around the pulley 14a. As shown in Fig. 7C, the rotating force is transmitted through the wire 15 tightened around the fitting member 12 to rotate the fitting member 12.

The operation of the thus-configured treatment instrument adaptor 6 and endoscope system 1 according to this embodiment will be described below.

To perform treatment in the body of the patient P using the endoscope system 1 according to this embodiment, the operator A inserts the insertion portion 5 of the endoscope 3 with a method which is the same as or similar to that for a conventional endoscope. The operator A operates the endoscope 3 to capture and display an image of the interior of the body on the monitor 25. While watching the monitor 25, the operator A inserts the insertion portion 5 of the endoscope 3 until the distal end of the insertion portion 5 approaches the affected area.

When the tip surface 5a of the insertion portion 5 is positioned near the affected area, the treatment instrument 8 is inserted from the proximal end of the channel 7 of the insertion portion 5 while the slider 16c of the slider mechanism 16 is placed in the release position shown in Fig. 4A, and then the distal end of the treatment instrument 8 protrudes from the tip surface 5a of the insertion portion 5. The through holes 11d aligned with the opening of the channel 7 are provided in the cap member 11 attached to the distal end of the insertion portion 5, and the fitting member 12 is disposed in the through holes 11d, with the inner hole 12d widened. The distal end of the treatment instrument 8 protruding from the opening of the channel 7 is fitted into the inner hole 12d of the fitting member 12 held in the through holes 11d.

As shown in Fig. 3, the treatment instrument 8 is protruded from the cap member 11 through the through holes 11d and is displayed on the monitor 25. The operator A can then operate the treatment instrument 8 to check the direction of the joints 10 of the treatment instrument 8 (the pivot direction of the end effector 9). If the operator A finds that the direction of the joints 10 is not the desired direction, the operator A operates the slider mechanism 16 to move the slider 16c to the lock position shown in Fig. 4B.

Thus, the wire 15 is tightened under tension to compress the fitting member 12, which then holds the treatment instrument 8 fitted in the inner hole 12d. The fitting member 12 and the treatment instrument 8 are coupled (engaged) to each other by friction. The operator A then rotates the pulley 14a of the rotating mechanism 14 by driving the motor to create an imbalance in the tensions on the wire 15 tightened around the fitting member 12. As shown in Fig. 7C, the fitting member 12 is rotated about the axis thereof.

As the fitting member 12 is rotated, the treatment instrument 8, which is coupled to the fitting member 12 by friction, is also rotated about the axis thereof to change the direction of the joints 10.

In this case, since the treatment instrument 8 is very thin and long, it is difficult to transmit a torsion, which is applied to the treatment instrument 8 at the proximal end of the insertion portion 5 at the outside of the body, to the distal end of the treatment instrument 8. However, this embodiment allows a rotating force to be transmitted to the vicinity of the distal end of the treatment instrument 8, thus providing an advantage of easily and reliably rotating the treatment instrument 8 about the axis thereof.

Thus, the direction of the joints 10 of the treatment instrument 8 can be changed in a state in which the treatment instrument 8 is placed in the body, and it is not necessary to remove the insertion portion 5 from the body to change the direction of the joints 10. This feature provides an advantage of reducing the time required for treatment and the burden on the patient.

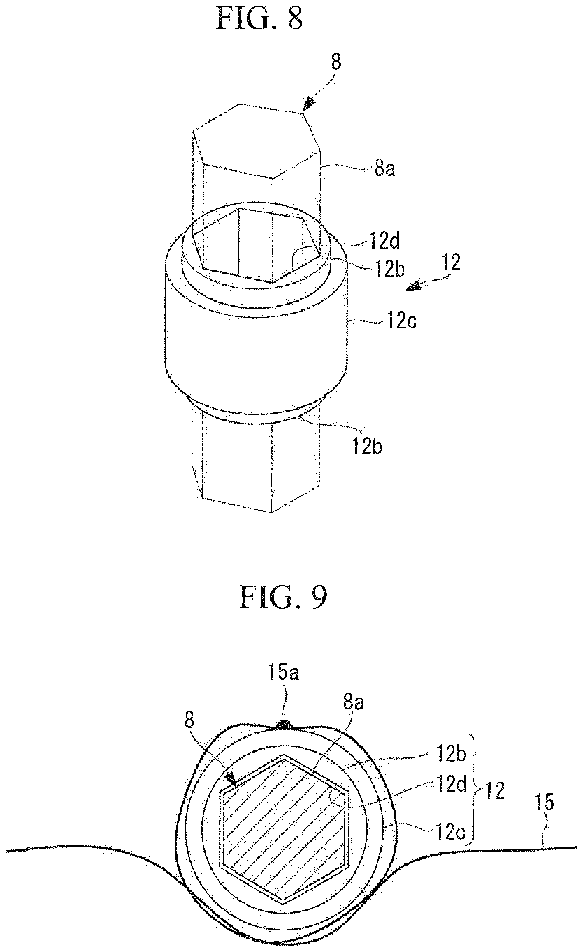

Although in this embodiment the treatment instrument 8 is coupled to and decoupled from the fitting member 12 by adjusting the tension applied to the wire 15 by the tightening mechanism 13, other mechanisms may be employed instead. For example, as shown in Fig. 8, a hexagonal pillar-like portion 8a may be provided at the distal end of the treatment instrument 8, and a hexagonal inner hole 12d may be formed in the fitting member 12 without the slit 12a.

In this case, the pillar-like portion 8a, which has a hexagonal cross-section, can be fitted into the inner hole 12d of the fitting member 12 and can be engaged with the fitting member 12 in the circumferential direction without compressing the inner hole 12d. This allows the rotating force of the fitting member 12 due to the tension on the wire 15 to be transmitted to the treatment instrument 8 to easily adjust the direction of the joints 10.

Instead of making the cross-sectional shape of the pillar-like portion 8a of the treatment instrument 8 hexagonal, the pillar-like portion 8a may have any noncircular cross-section such as a polygonal or oval cross-section. In this case, the inner hole 12d of the fitting member 12 may have a complementary or partially complementary cross-section matching the cross-section of the pillar-like portion 8a.

In this case, it is not necessary to tighten the wire 15 around the fitting member 12. Therefore, as shown in Fig. 9, the wire 15 may include a fixed portion 15a welded or bonded to the fitting member 12. The wire 15 does not need to be a single wire 15, but may be a pair of separate wires.

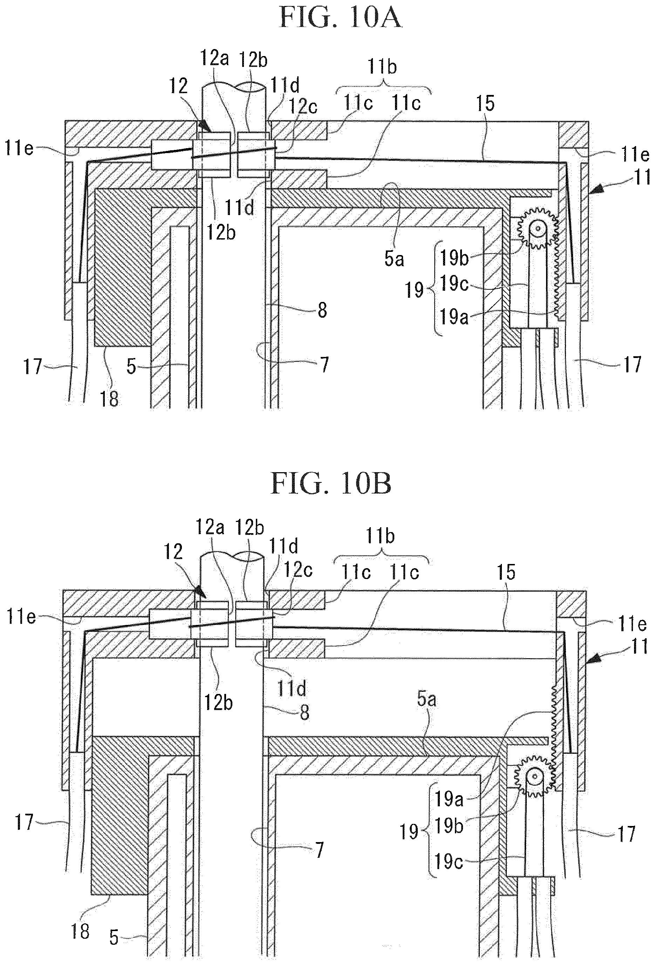

If the wire 15 is used to decrease the inner diameter of the inner hole 12d of the fitting member 12, as shown in Fig. 10, the treatment instrument adaptor 6 may include a fixed member 18 fixed to the distal end of the insertion portion 5 and supporting the cap member 11 so as to be movable in the longitudinal axis direction of the insertion portion 5, and a moving mechanism 19 that moves the cap member 11 relative to the fixed member 18.

In this case, as shown in Fig. 10A, when the moving mechanism 19 is operated while tension is applied to the wire 15 to hold the treatment instrument 8 with the fitting member 12, and when the cap member 11 is moved forward relative to the fixed member 18 as shown in Fig. 10B, the treatment instrument 8 can be moved forward together with the cap member 11.

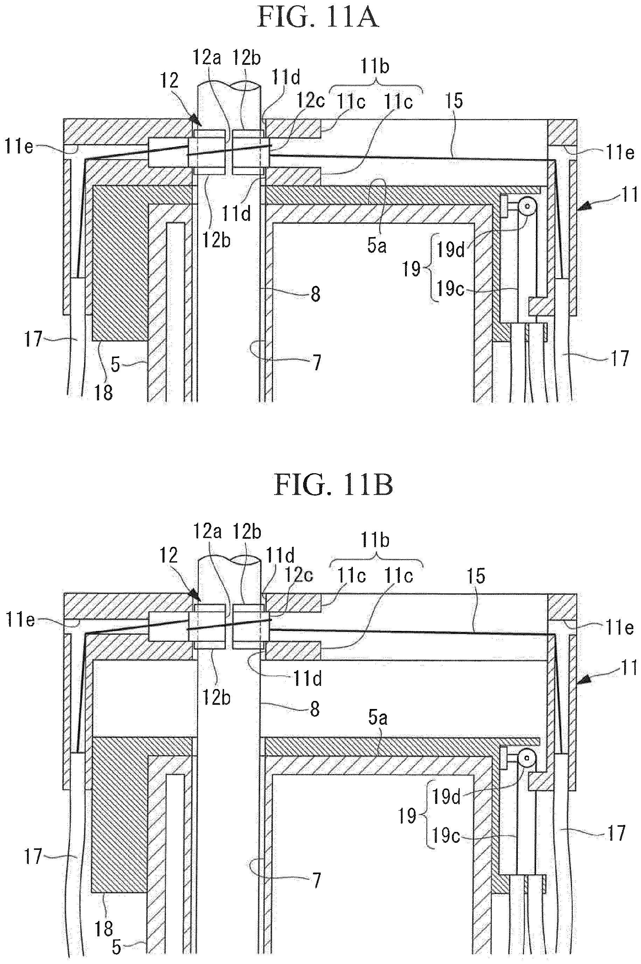

As shown in Fig. 10, as the moving mechanism 19, a type of the mechanism which has a rack gear 19a and a pinion gear 19b meshing with each other, and a wire 19c that rotates the pinion gear 19b may be employed. As shown in Figs. 11A and 11B, a type of the mechanism whose wire 19c which runs around a pulley 19d is secured to part of the cap member 11 may be employed.

Although the fitting member 12 has a C-shaped cross-section with the slit 12a so that the inner hole 12d can be expanded and compressed, the fitting member 12 may instead have an annular cross-section without the slit 12a and may be made of an elastic material that allows the diameter of the inner hole 12d to be decreased as the wire 15 is tightened.

Although the wire 15 is used as an example driving-force transmitting member in the aforementioned embodiment, it may be replaced with a flexible member that can transmit tension, such as a cable or belt.

Although the treatment instrument adaptor 6 is attached to the endoscope 3 in this embodiment, it may instead be attached to a flexible insertion portion which is equipped with an external channel and which is provided in an endoscope 3.

### {Reference Signs List}

- 1: endoscope system (surgical manipulator system)
- 3: endoscope
- 5: insertion portion
- 6: treatment instrument adaptor
- 7: channel
- 8: treatment instrument
- 9: end effector
- 11: cap member
- 11d: through hole
- 12: fitting member
- 12a: slit
- 12d: inner hole
- 13: tightening mechanism
- 14: rotating mechanism
- 15: wire (driving-force transmitting member)
- 18: fixed member
- 19: moving mechanism

## Claims

1. A treatment instrument adaptor comprising:
a cap member that is attached to a distal end of a flexible insertion portion having a channel and that has a through hole located at a position which is aligned with an opening of the channel;
a substantially tubular fitting member that is attached to the through hole of the cap member so as to be rotatable about an axis of the through hole and so as not to be movable along the axis and that has an inner hole into which a treatment instrument guided through the channel is fitted and with which the treatment instrument is engaged in a circumferential direction; and
a rotating mechanism including a driving-force transmitting member that is wound around the fitting member and that rotates the fitting member about the axis using tension.

2. The treatment instrument adaptor according to claim 1, wherein the fitting member is made of an elastic material that allows an inner diameter of the inner hole to be changed, and the inner diameter of the inner hole is configured to be decreased to engage the inner hole with the treatment instrument in a state in which the treatment instrument is placed in the inner hole.

3. The treatment instrument adaptor according to claim 2, wherein the fitting member has a C-shaped cross-section with a slit provided at a part of the circumferential direction.

4. The treatment instrument adaptor according to claim 2 or 3, further comprising a tightening mechanism that applies tension to the driving-force transmitting member to decrease the inner diameter of the inner hole of the fitting member.

5. The treatment instrument adaptor according to any one of claims 2 to 4, further comprising:
a fixed member fixed to the insertion portion; and
a moving mechanism that moves the cap member relative to the fixed member in a longitudinal axis direction of the insertion portion.

6. The treatment instrument adaptor according to claim 1, wherein the treatment instrument is provided with a pillar-like fitting portion having a noncircular cross-section, and the inner hole of the fitting member has a cross-section that allows the pillar-like fitting portion to be fitted into the inner hole and to be engaged with the inner hole of the fitting member in the circumferential direction.

7. A surgical manipulator system comprising:
a treatment instrument adaptor according to any one of Claims 1 to 6;
an endoscope to which the cap member of the treatment instrument adaptor is attached at a distal end of the endoscope; and
a treatment instrument that is guided through the channel of the insertion portion of the endoscope and that includes an end effector that is protruded from the distal end of the insertion portion through the inner hole of the fitting member.
